# EUROPEAN PATENT APPLICATION

(11) **EP 4 173 628 A1**
(43) Date of publication of application: **03.05.2023**
(21) Application number: 21829814.9
(22) Date of filing: 28.01.2021
(51) Int. Cl.: A61K 35/28, A61K 35/32, A61P 15/08

(54) **TESTICULAR FUNCTION IMPROVING AGENT AND TESTICULAR FUNCTION IMPROVING METHOD**

(30) Priority: 26.06.2020 JP 2020110422
(71) Applicant: Dexon Pharmaceuticals Inc., Tokyo, 103-0027 (JP)
(72) Inventor: KOGA Shoji, Tokyo 102-0082 (JP)
(74) Representative: Godemeyer Blum Lenze Patentanwälte Partnerschaft mbB - werkpatent
(86) International application number: PCT/JP2021/002962
(87) International publication number: WO 2021/260986

(57) **Abstract**

A testicular function-improving agent which contains microparticles derived from a culture supernatant of dental pulp-derived stem cells, adipose-derived stem cells or immortalized stem cells thereof and in which the improvement of a testicular function is improvement of the ability of a testis itself to form sperm, improvement of the function of sperm themselves obtained from a testis, improvement of the function of sperm obtained from a testis of acting on a female, rejuvenation of any of the functions that has aged with aging or activation of sperm through addition to semen taken out of the body of an animal can significantly improve a testicular function of an animal.

## Description

### Technical Field

The present invention relates to a testicular function-improving agent and a testicular function-improving method.

### Background Art

As a method for improving or treating infertility or a method for stabilizing artificial insemination of a domestic animal, a method for activating sperm using mesenchymal stem cells has been known (see PTL 1).

PTL 1 describes a sperm activator whose active ingredient is a disrupted product of one or more cells selected from the group consisting of adipose tissue-derived stem cells, dental pulp-derived stem cells, bone marrow-derived stem cells and umbilical cord blood-derived stem cells. PTL 1 also discloses that the main component of the active ingredient of the disrupted product of dental pulp-derived stem cells or the like is HSP90α, which is one of heat shock proteins (HSPs).

### Citation List

### Patent Literature

PTL 1: WO2018/038180

### Summary of Invention

### Technical Problem

PTL 1 only discloses that, when the sperm activator is added to semen taken out of the body of an animal, the motility or the like of sperm can be maintained. Thus, PTL 1 does not suggest a sperm activator which is directly administered to an animal to improve a testicular function.

In this regard, because the sperm activator described in PTL 1 is produced by collecting a cell population of mesenchymal stem cells and then disrupting the cells by freeze-thaw treatment or the like, direct administration thereof to an animal is believed to be difficult due to the high impurity content in the first place.

A problem that the invention is to solve is to provide a testicular function-improving agent which can significantly improve a testicular function of an animal.

The "testicular function" here refers to the function of a testis of producing sperm, and the function of the sperm means the normal reproductive function which enables normal pregnancy, fetal growth and birth.

### Solution to Problem

In the invention, it has been found that a testicular function of an animal can be significantly improved using microparticles derived from a culture supernatant of dental pulp-derived stem cells or a culture supernatant of adipose-derived stem cells (or a culture supernatant of immortalized stem cells thereof), and the problem has been thus solved.

In this regard, PTL 1 relates to a sperm activator which is added to semen taken out of the body of an animal and thus focuses on a heat shock protein as the active ingredient, and a dental pulp-derived stem cell-derived or adipose-derived stem cell-derived culture supernatant or microparticles such as exosomes have not been focused on.

The constitution of the invention as specific means for solving the problem and preferable aspects of the invention are described below.
[1] A testicular function-improving agent containing microparticles derived from a culture supernatant of dental pulp-derived stem cells, adipose-derived stem cells or immortalized stem cells thereof,
   wherein the improvement of a testicular function is improvement of the ability of a testis itself to form sperm, improvement of the function of sperm themselves obtained from a testis, improvement of the function of sperm obtained from a testis of acting on a female, rejuvenation of any of the functions that has aged with aging or activation of sperm through addition to semen taken out of the body of an animal.
[2] The testicular function-improving agent described in [1], wherein the microparticles are exosomes.
[3] The testicular function-improving agent described in [1] or [2]
   which does not contain the dental pulp-derived stem cells, the adipose-derived stem cells and the immortalized stem cells,
   does not contain MCP-1 and
   does not contain Siglec-9.
[4] The testicular function-improving agent described in any one of [1] to [3] which is an agent for improving the spermatogenic ability of a testis.
[5] The testicular function-improving agent described in any one of [1] to [3] which is a fertility-improving agent.
[6] The testicular function-improving agent described in any one of [1] to [3] which is a deformity suppressor.
[7] The testicular function-improving agent described in any one of [1] to [6], wherein the microparticles are microparticles purified from the culture supernatant.
[8] The testicular function-improving agent described in any one of [1] to [7] which contains 0.5×10⁸ particles or more of the microparticles.
[9] The testicular function-improving agent described in any one of [1] to [8], wherein the dental pulp-derived stem cells or the adipose-derived stem cells are human dental pulp-derived stem cells or human adipose-derived stem cells.
[10] The testicular function-improving agent described in any one of [1] to [8], wherein the dental pulp-derived stem cells or the adipose-derived stem cells are dental pulp-derived stem cells of a nonhuman animal or adipose-derived stem cells of a nonhuman animal.
[11] A method for improving a testicular function including a step of administering the testicular function-improving agent described in any one of [1] to [10] to an animal.
[12] The method for improving a testicular function described in [11], characterized by
   improving the spermatogenic ability of a testis of the animal,
   improving the fertility of the animal or
   suppressing deformity of offspring of the animal.
[13] The method for improving a testicular function described in [11] or [12], wherein the testicular function-improving agent is injected to a testis of the animal.
[14] The method for improving a testicular function described in any one of [11] to [13], wherein the dosage of the microparticles per testis of the animal is 0.5×10⁸ to 2×10⁸ particles/testis.

### Advantageous Effects of Invention

According to the invention, a testicular function-improving agent which can significantly improve a testicular function of an animal can be provided.

### Brief Description of Drawings

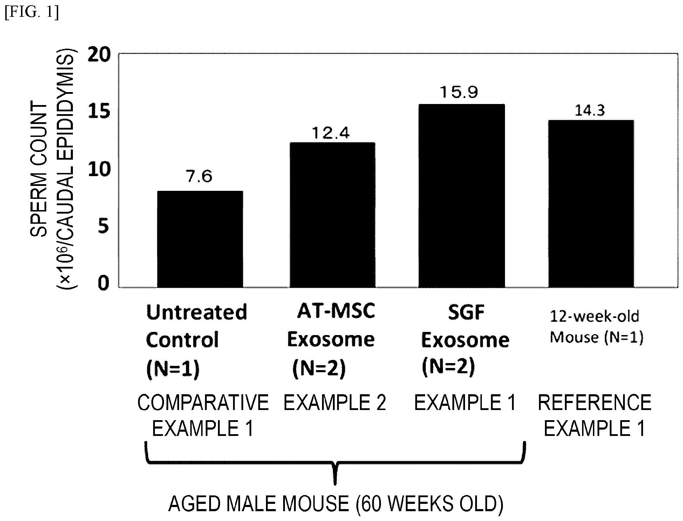

[Fig. 1] Fig. 1 is a bar chart comparing the sperm counts among the aged male mice to which the testicular function-improving agent of Example 1 (SGF exosome) or the adipose tissue-derived exosomes of Example 2 (AT-MSC exosome) were administered, the aged male mouse of Comparative Example 1 without the exosome administration (Untreated Control) and the young male mouse of Reference Example 1.

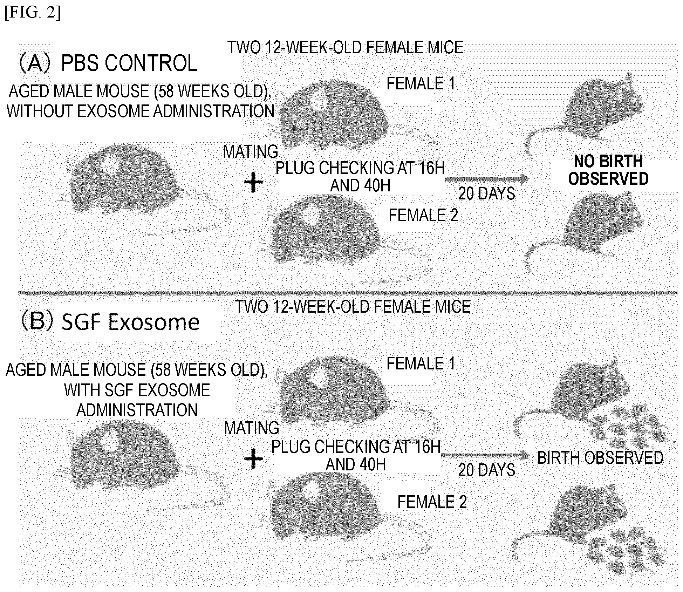

[Fig. 2] Fig. 2(A) shows the scheme of the mating test and the birth test of Comparative Examples 11 and 12 (PBS control), in which physiological saline was administered to the testes of aged male mice. Fig. 2(B) shows the scheme of the mating test and the birth test of Examples 11 and 12, in which the testicular function-improving agent of Example 1 (SGF exosome) was administered to the testes of aged male mice.

### Description of Embodiments

The invention is explained in detail below. The constituent features described below are sometimes explained based on typical embodiments or specific examples, but the invention should not be limited to the embodiments. In the specification, a numerical range expressed using "to" means a range including the values before and after "to" as the lower limit and the upper limit.

### [Testicular Function-Improving Agent]

The testicular function-improving agent of the invention contains microparticles derived from a culture supernatant of dental pulp-derived stem cells, adipose-derived stem cells or immortalized stem cells thereof (also called a culture supernatant of dental pulp-derived stem cells or adipose-derived stem cells below).

The improvement of a testicular function is improvement of the ability of a testis itself to form sperm, improvement of the function of sperm themselves obtained from a testis, improvement of the function of sperm obtained from a testis of acting on a female, rejuvenation of any of the functions that has aged with aging or activation of sperm through addition to semen taken out of the body of an animal.

Due to the constitution, a testicular function-improving agent which can significantly improve a testicular function of an animal can be provided.

Although the immortalized stem cells are sometimes omitted in the following descriptions, the explanation of the dental pulp-derived stem cells or the adipose-derived stem cells also includes immortalized stem cells thereof.

Preferable aspects of the invention are explained below.

### <Microparticles>

In the invention, microparticles derived from a culture supernatant of dental pulp-derived stem cells or adipose-derived stem cells are used. The microparticles are derived from dental pulp-derived stem cells or adipose-derived stem cells, for example, through secretion, emergence, dispersion or the like from the dental pulp-derived stem cells or the adipose-derived stem cells and are exuded or released or fall into a cell culture medium. Thus, the microparticles are contained in a culture supernatant of dental pulp-derived stem cells or adipose-derived stem cells.

When the testicular function-improving agent of the invention is used, the microparticles derived from a culture supernatant of dental pulp-derived stem cells, adipose-derived stem cells or immortalized stem cells thereof may be contained in the culture supernatant or may be purified from the culture supernatant. That is, the culture supernatant of dental pulp-derived stem cells, adipose-derived stem cells or immortalized stem cells thereof may be used as the testicular function-improving agent of the invention, or a composition containing microparticles purified from the culture supernatant may be used as the testicular function-improving agent of the invention.

In the invention, the microparticles are preferably microparticles purified from the culture supernatant.

### (Characteristics of Microparticles)

The microparticles are preferably of at least one kind selected from the group consisting of exosomes, microvesicles, membrane particles, membrane vesicles, ectosomes, exovesicles and microvesicles, more preferably exosomes.

The diameter of the microparticles is preferably 10 to 1000 nm, more preferably 30 to 500 nm, particularly preferably 50 to 150 nm. Moreover, molecules called tetraspanins such as CD9, CD63 and CD81 are desirably on the surface of the microparticles, and the molecules may be those of CD9 alone, CD63 alone, CD81 alone or any combination of two or three thereof.

A preferable aspect in which exosomes are used as the microparticles is sometimes explained below, but the microparticles of the invention are not limited to exosomes.

The exosomes are preferably extracellular vesicles which are released from cells during fusion of plasma membrane and multivesicular bodies.

The surface of the exosomes preferably contains a lipid and a protein derived from the cell membrane of dental pulp-derived stem cells or adipose-derived stem cells (or immortalized stem cells thereof).

The exosomes preferably contain, in their inside, an intracellular substance of dental pulp-derived stem cells or adipose-derived stem cells such as nucleic acids (microRNAs, messenger RNAs, DNAs and the like) and proteins.

The exosomes are known to be used for communication among cells through transportation of genetic information from a cell to another cell. The exosomes can be tracked easily and can be targeted at a specific region.

### (Microparticle Content)

The microparticle content of the testicular function-improving agent of the invention is not particularly restricted. The testicular function-improving agent of the invention preferably contains 0.5×10⁸ particles or more, more preferably 1.0×10⁸ particles or more, particularly preferably 2.0×10⁸ particles or more of the microparticles.

The concentration of the microparticles contained in the testicular function-improving agent of the invention is not particularly restricted. The testicular function-improving agent of the invention preferably contains the microparticles at 0.01×10⁸ particles/mL or more, more preferably at 0.1×10⁸ particles/mL or more, particularly preferably 1.0×10⁸ particles/mL or more.

### (Culture Supernatant of Dental Pulp-Derived Stem Cells or Adipose-Derived Stem Cells)

The culture supernatant of dental pulp-derived stem cells or adipose-derived stem cells is not particularly restricted.

The culture supernatant of dental pulp-derived stem cells or adipose-derived stem cells preferably does not substantially contain serum. For example, the serum content of the culture supernatant of dental pulp-derived stem cells or adipose-derived stem cells is preferably 1 mass% or less, more preferably 0.1 mass% or less, particularly preferably 0.01 mass% or less.

### - Dental Pulp-Derived Stem Cells or Adipose-Derived Stem Cells -

The dental pulp-derived stem cells or the adipose-derived stem cells may be derived from a human or from a nonhuman animal. The nonhuman animal may be the same as the animal (species) as the subject of the administration of the testicular function-improving agent described below and is preferably a mammal.

The dental pulp-derived stem cells used for the culture supernatant are not particularly restricted. Dental pulp stem cells from exfoliated deciduous teeth, deciduous dental pulp stem cells obtained by another method and permanent dental pulp stem cells (DPSCs) can be used. In addition to human deciduous dental pulp stem cells and human permanent dental pulp stem cells, dental pulp-derived stem cells derived from a nonhuman animal such as pig deciduous dental pulp stem cells can be used.

The dental pulp-derived stem cells (or the adipose-derived stem cells) can produce, in addition to exosomes, vascular endothelial growth factors (VEGFs), hepatocyte growth factors (HGFs), insulin-like growth factors (IGFs), platelet-derived growth factors (PDGFs), transforming growth factors-beta (TGF-β)-1 and -3, TGF-a, KGF, HBEGF, SPARC, other growth factors and various cytokines such as chemokine. Many other bioactive substances can also be produced.

In the invention, the dental pulp-derived stem cells used for the culture supernatant of dental pulp-derived stem cells particularly preferably contain many proteins, and deciduous dental pulp stem cells are preferably used. That is, in the invention, a culture supernatant of deciduous dental pulp stem cells (SGF) is preferably used.

The adipose-derived stem cells used for the culture supernatant are not particularly restricted. As the adipose-derived stem cells, somatic stem cells contained in any adipose tissue can be used. In addition to human adipose-derived stem cells, adipose-derived stem cells derived from a nonhuman animal such as pig adipose stem cells can be used. As the adipose-derived stem cells, for example, adipose-derived stem cells prepared by the method described in [0023] to [0041] of WO2018/038180 can be used, and the contents of the publication are incorporated in the present specification by reference.

The dental pulp-derived stem cells or the adipose-derived stem cells used in the invention may be natural cells or genetically modified cells as long as the intended treatment can be achieved.

In particular, in the invention, immortalized stem cells of dental pulp-derived stem cells or adipose-derived stem cells can be used. Using immortalized stem cells, which are capable of proliferating substantially infinitely, the amounts and the compositions of the biological factors contained in the culture supernatant of the stem cells can be stabilized over a long period of time. The immortalized stem cells of dental pulp-derived stem cells or adipose-derived stem cells are not particularly restricted. The immortalized stem cells are preferably non-tumor immortalized stem cells. The immortalized stem cells of dental pulp-derived stem cells or adipose-derived stem cells can be prepared by adding one of the following small molecule compounds (inhibitors) or any combination thereof to dental pulp-derived stem cells or adipose-derived stem cells and culturing the cells.

TGFβ receptor inhibitors are not particularly limited as long as the TGFβ receptor inhibitors have the action of inhibiting the function of transforming growth factor (TGF) β receptor, and examples thereof include 2-(5-benzo[1,3]dioxol-4-yl-2-tert-butyl-1H-imidozol-4-yl)-6-methylpyridine, 3-(6-methylpyridin-2-yl)-4-(4-quinolyl)-1-phenylthiocarbamoyl-1H-pyrazole (A-83-01), 2-(5-chloro-2-fluorophenyl)pteridin-4-yl)pyridin-4-ylamine (SD-208), 3-(pyridin-2-yl)-4-(4-quinonyl)]-1H-pyrazole and 2-(3-(6-methylpyridin-2-yl)-1H-pyrazol-4-yl)-1,5-naphthyridine (all are from Merck), SB431542 (Sigma-Aldrich) and the like. A-83-01 is preferable.

ROCK inhibitors are not particularly limited as long as the ROCK inhibitors have the action of inhibiting the function of Rho-binding kinase. Examples of the ROCK inhibitors include GSK269962A (Axonmedchem), Fasudil hydrochloride (Tocris Bioscience), Y-27632 and H-1152 (both are from FUJIFILM Wako Pure Chemical Corporation) and the like. Y-27632 is preferable.

GSK3 inhibitors are not particularly limited as long as GSK-3 (glycogen synthase kinase 3) is inhibited, and examples thereof include A 1070722, BIO and BIO-acetoxime (all are from TOCRIS) and the like.

MEK inhibitors are not particularly limited as long as the MEK inhibitors have the action of inhibiting the function of MEK (MAP kinase-ERK kinase), and examples thereof include AZD6244, CI-1040 (PD184352), PD0325901, RDEA119 (BAY86-9766), SL327 and U0126-EtOH (all are from Selleck), PD98059, U0124 and U0125 (all are from Cosmo Bio Co., Ltd.) and the like.

When the testicular function-improving agent of the invention is used for regenerative medicine, due to the requirements of the Act on the Safety of Regenerative Medicine, the testicular function-improving agent containing microparticles derived from a culture supernatant of dental pulp-derived stem cells or adipose-derived stem cells does not contain other somatic stem cells than the dental pulp-derived stem cells or the adipose-derived stem cells. When the testicular function-improving agent of the invention is used in a technical field other than regenerative medicine, however, the testicular function-improving agent may contain mesenchymal stem cells other than the dental pulp-derived stem cells or the adipose-derived stem cells or other somatic stem cells but preferably does not contain such cells.

The mesenchymal stem cells other than the dental pulp-derived stem cells or the adipose-derived stem cells include bone marrow-derived stem cells, umbilical cord-derived stem cells and umbilical cord blood-derived stem cells but are not limited to these types of cells.

Examples of the somatic stem cells other than the mesenchymal stem cells include stem cells derived from the dermal system, the digestive system, the myeloid system, the nerve system and the like, but the somatic stem cells are not limited to the examples. Examples of the somatic stem cells of the dermal system include epithelial stem cells, hair follicle stem cells and the like. Examples of the somatic stem cells of the digestive system include pancreatic (general) stem cells, hepatic stem cells and the like. Examples of the somatic stem cells of the myeloid system (except for the mesenchymal stem cells) include hematopoietic stem cells and the like. Examples of the somatic stem cells of the nerve system include neural stem cells, retinal stem cells and the like.

The testicular function-improving agent of the invention may contain stem cells other than somatic stem cells but preferably does not contain such stem cells. The stem cells other than somatic stem cells include embryonic stem cells (ES cells), induced pluripotent stem cells (iPS cells) and embryonal carcinoma cells (EC cells).

### - Preparation Method of Culture Supernatant of Dental Pulp-Derived Stem Cells or Adipose-Derived Stem Cells -

The method for preparing the culture supernatant of dental pulp-derived stem cells or adipose-derived stem cells is not particularly restricted, and a conventional method can be used.

The culture supernatant of dental pulp-derived stem cells or adipose-derived stem cells is a culture solution obtained by culturing dental pulp-derived stem cells or adipose-derived stem cells and does not contain the cells themselves. For example, a culture supernatant which can be used for the invention can be obtained by culturing dental pulp-derived stem cells or adipose-derived stem cells and then separating and removing the cell components. A culture supernatant which has been appropriately subjected to various treatments (for example, centrifugation, concentration, solvent substitution, dialysis, freezing, drying, lyophilization, dilution, desalination, preservation or the like) may also be used.

The dental pulp-derived stem cells or the adipose-derived stem cells for obtaining the culture supernatant of dental pulp-derived stem cells or adipose-derived stem cells can be screened by a normal method and can be screened based on the size and the morphology of the cells or as adherent cells. The dental pulp-derived stem cells can be screened from dental pulp cells collected from an exfoliated deciduous tooth or a permanent tooth as adherent cells or passaged cells thereof. The adipose-derived stem cells can be screened from adipose cells collected from adipose tissue as adherent cells or passaged cells thereof. The culture supernatant of dental pulp-derived stem cells or adipose-derived stem cells used can be a culture supernatant obtained by culturing screened stem cells.

The "culture supernatant of dental pulp-derived stem cells or adipose-derived stem cells" is preferably a culture solution which does not contain the cells themselves obtained by culturing the dental pulp-derived stem cells or the adipose-derived stem cells. The culture supernatant of dental pulp-derived stem cells or adipose-derived stem cells used in the invention preferably does not contain any cells (regardless of the kinds of cells) as a whole in an aspect. Due to the feature, the testicular function-improving agent of the aspect is clearly distinguished of course from the dental pulp-derived stem cells or the adipose-derived stem cells themselves and also from various compositions containing dental pulp-derived stem cells or adipose-derived stem cells. A typical example of the aspect is a composition which does not contain dental pulp-derived stem cells or adipose-derived stem cells and which is composed only of a culture supernatant of dental pulp-derived stem cells or adipose-derived stem cells.

The culture supernatant of dental pulp-derived stem cells used in the invention may contain a culture supernatant of both deciduous dental pulp-derived stem cells and adult dental pulp-derived stem cells. The culture supernatant of dental pulp-derived stem cells used in the invention preferably contains a culture supernatant of deciduous dental pulp-derived stem cells as an active ingredient, more preferably in an amount of 50 mass% or more, preferably 90 mass% or more. The culture supernatant of dental pulp-derived stem cells used in the invention is particularly preferably a composition composed only of a culture supernatant of deciduous dental pulp-derived stem cells.

A basal medium, a basal medium supplemented with serum and the like or the like can be used for the culture solution of dental pulp-derived stem cells or adipose-derived stem cells for obtaining the culture supernatant. Examples of the basal medium which can be used include, in addition to Dulbecco's modified Eagle's medium (DMEM), Iscove's modified Dulbecco's medium (IMDM) (GIBCO and the like), Ham's F12 medium (SIGMA, GIBCO and the like), RPMI1640 medium and the like. Examples of the component that can be added to the medium include serum (fetal bovine serum, human serum, sheep serum and the like), serum replacements (knockout serum replacement (KSR) and the like), bovine serum albumin (BSA), antibiotics, vitamins and minerals.

In order to prepare "a culture supernatant of dental pulp-derived stem cells or adipose-derived stem cells" which does not contain serum, however, a serum-free medium should be used throughout the whole process or for the last passaging culture or the last passaging culture steps. For example, when dental pulp-derived stem cells or adipose-derived stem cells are cultured in a medium which does not contain serum (a serum-free medium), a culture supernatant of dental pulp-derived stem cells or adipose-derived stem cells which does not contain serum can be prepared. When passaging culture is conducted once or two or more times and when the last passaging culture or the last passaging culture steps are conducted in a serum-free medium, a culture supernatant of dental pulp-derived stem cells or adipose-derived stem cells which does not contain serum can also be obtained. Moreover, a culture supernatant of dental pulp-derived stem cells or adipose-derived stem cells which does not contain serum can also be obtained by removing serum from the recovered culture supernatant using dialysis, solvent substitution using a column or the like.

For culturing the dental pulp-derived stem cells or the adipose-derived stem cells for obtaining the culture supernatant, generally used conditions can be applied as they are. The method for preparing a culture supernatant of the dental pulp-derived stem cells or the adipose-derived stem cells can be the same as the cell culture method described below except that the isolation and screening steps of the stem cells are appropriately adjusted to the type of the stem cells. One skilled in the art can appropriately isolate and screen dental pulp-derived stem cells or adipose-derived stem cells according to the type of the dental pulp-derived stem cells or the adipose-derived stem cells.

In order to produce a large amount of a specific kind of exosomes which contributes to improvement of a testicular function, special conditions may be applied for culturing the dental pulp-derived stem cells or the adipose-derived stem cells. The special conditions may be, for example, low-temperature conditions, low-oxygen conditions, microgravity conditions, conditions of coculturing with any stimulant or the like.

### - Other Components Contained in Culture Supernatant of Dental Pulp-Derived Stem Cells or Adipose-Derived Stem Cells -

The culture supernatant of dental pulp-derived stem cells or adipose-derived stem cells used for preparing exosomes in the invention may contain another component in addition to the culture supernatant of dental pulp-derived stem cells or adipose-derived stem cells but preferably does not substantially contain any other component.

The culture supernatant of dental pulp-derived stem cells or adipose-derived stem cells may be stored after an additive used for preparing exosomes is added to the culture supernatant.

### (Purification of Microparticles)

Microparticles can be purified from the culture supernatant of dental pulp-derived stem cells or adipose-derived stem cells.

The purification of the microparticles is preferably separation of a fraction containing the microparticles from the culture supernatant of dental pulp-derived stem cells or adipose-derived stem cells, more preferably isolation of the microparticles.

The microparticles can be isolated by separation from non-associated components based on the characteristics of the microparticles. For example, the microparticles can be isolated based on the molecular weight, the size, the morphology, the composition or the biological activity.

In the invention, the microparticles can be purified by centrifuging the culture supernatant of dental pulp-derived stem cells or adipose-derived stem cells and collecting a specific fraction containing a large amount of the microparticles (for example, precipitates) obtained by the centrifugation. Unnecessary components (insoluble components) in a faction other than the predetermined fraction may be removed. The solvent, the dispersion medium and the unnecessary components do not have to be removed completely from the testicular function-improving agent. An example of the centrifugation conditions is 100 to 20000 g for 1 to 30 minutes.

In the invention, the microparticles can be purified by subjecting the culture supernatant of dental pulp-derived stem cells or adipose-derived stem cells or a centrifuged product thereof to filtration. Unnecessary components can be removed through the filtration. Moreover, when a filter membrane having an appropriate pore size is used, the removal of the unnecessary components and sterilization can be conducted simultaneously. The material, the pore size and the like of the filter membrane used for the filtration are not particularly limited. The filtration can be achieved by a known method using a filter membrane having an appropriate molecular weight or an appropriate size cut-off. Because exosomes can be easily fractionated, the pore size of the filter membrane is preferably 10 to 1000 nm, more preferably 30 to 500 nm, particularly preferably 50 to 150 nm.

In the invention, the culture supernatant of dental pulp-derived stem cells or adipose-derived stem cells, a centrifuged product thereof or a filtered product thereof can be separated further using separation means such as RAM chromatography. For example, high-performance liquid chromatography (HPLC) using any of various columns can be used. The column which can be used is a size exclusion column or a binding column.

In order to track the microparticles (or the activity thereof) in the fraction at each treatment stage, one or more characteristics or the biological activity of the microparticles can be used. For example, in order to track the microparticles, light scattering, refractive index, dynamic light scattering or UV-VIS detector can be used. Alternatively, in order to track the activity in each fraction, a therapeutic activity such as testicular function-improving activity can be used.

As the method for purifying the microparticles, the method described in [0034] to [0064] of JP-T-2019-524824 (the term "JP-T" as used herein means a published Japanese translation of a PCT patent application) may be used, and the contents of the publication are incorporated in the present specification by reference.

### <Other Components>

The testicular function-improving agent of the invention may contain another component in addition to the microparticles depending on the species of the animal as the subject of the administration or the purpose as long as the effects of the invention are not impaired. Examples of the other component include nutritional components, antibiotics, cytokines, protectants, carriers, excipients, disintegrating agents, buffers, emulsifiers, suspending agents, soothing agents, stabilizers, preservatives, antiseptics and the like.

Examples of the nutritional components include fatty acids, vitamins and the like.

Examples of the antibiotics include penicillin, streptomycin, gentamicin and the like.

The carriers may be materials known as pharmaceutically acceptable carriers.

The testicular function-improving agent of the invention may be the microparticles themselves or a pharmaceutical composition further containing a pharmaceutically acceptable carrier or excipient or the like. The purpose of the pharmaceutical composition is to promote administration of the microparticles to the subject animal of the administration.

The pharmaceutically acceptable carrier is preferably a carrier (including a diluent) which does not cause significant irritation in the subject animal of the administration and which does not inhibit the biological activity and the characteristics of the administered composition. Examples of the carrier include propylene glycol, (physiological) saline, an emulsion, a buffer, a medium such as DMEM and RPMI and a low-temperature preservation medium containing a component for removing free radicals.

The testicular function-improving agent of the invention preferably does not contain specific substances.

For example, the testicular function-improving agent of the invention preferably does not contain dental pulp-derived stem cells or adipose-derived stem cells.

Moreover, the testicular function-improving agent of the invention preferably does not contain MCP-1. In this regard, however, cytokines other than MCP-1 may be contained. Examples of the other cytokines include those described in [0014] to [0020] of JP-A-2018-023343 and the like.

The testicular function-improving agent of the invention preferably does not contain Siglec-9. In this regard, however, sialic acid-binding immunoglobulin-like lectins other than Siglec-9 may be contained.

The testicular function-improving agent of the invention preferably does not substantially contain serum (fetal bovine serum, human serum, sheep serum or the like). Moreover, the testicular function-improving agent of the invention preferably does not substantially contain the conventional serum replacements such as knockout serum replacement (KSR).

The amounts (solid amounts) of the other components in the testicular function-improving agent of the invention are each preferably 1 mass% or less, more preferably 0.1 mass% or less, particularly preferably 0.01 mass% or less.

### <Production Method of Testicular Function-Improving Agent>

The method for producing the testicular function-improving agent of the invention is not particularly restricted.

A culture supernatant of dental pulp-derived stem cells or adipose-derived stem cells prepared by the above method or a commercially purchased culture supernatant of dental pulp-derived stem cells or adipose-derived stem cells may be used as a testicular function-improving agent.

Moreover, a testicular function-improving agent may be prepared by preparing a culture supernatant of dental pulp-derived stem cells or adipose-derived stem cells by the above method and subsequently purifying microparticles from the culture supernatant of dental pulp-derived stem cells or adipose-derived stem cells. Alternatively, a testicular function-improving agent may be prepared by purifying microparticles from a commercially purchased culture supernatant of dental pulp-derived stem cells or adipose-derived stem cells. A testicular function-improving agent may also be prepared by receiving a composition containing a culture supernatant of dental pulp-derived stem cells or adipose-derived stem cells which has been disposed of (or by appropriately purifying such a composition) and purifying microparticles from the composition.

The final form of the testicular function-improving agent of the invention is not particularly restricted. For example, the testicular function-improving agent of the invention may be in a form in which the microparticles are packed in a container with a solvent or a dispersion medium, a form in which the microparticles are formed into gel with gel and packed in a container, a form in which the microparticles are solidified by freezing and/or drying and formulated or packed in a container or another form. Examples of the container include a tube, a centrifuge tube, a bag or the like which is suitable for cryopreservation. The freezing temperature can be, for example, -20°C to - 196°C.

### <Applications of Testicular Function-Improving Agent>

The testicular function-improving agent of the invention can improve a testicular function. The testicular functions include, in addition to the ability of a testis itself to form sperm (increasing of the sperm count or the like) and other functions of a testis itself (increasing of a male hormone or the like), the function of sperm themselves obtained from a testis (improvement of the motility, the motility rate or the lifespan, reduction in genetic abnormality or the like) and the function of sperm obtained from a testis of acting on a female (fertility-improving ability, recurrent miscarriage-improving ability such as deformity-suppressing ability or the like). The testicular function-improving agent also has the effect of rejuvenating a testicular function which has aged with aging, and the rejuvenation applies to all the above items.

In the invention, the improvement of a testicular function is improvement of the ability of a testis itself to form sperm, improvement of the function of sperm themselves obtained from a testis, improvement of the function of sperm obtained from a testis of acting on a female, rejuvenation of any of the functions that has aged with aging or activation of sperm through addition to semen taken out of the body of an animal. That is, the testicular functions which can be improved with the testicular function-improving agent of the invention are the ability of a testis itself to form sperm (increasing of the sperm count or the like), the function of sperm themselves obtained from a testis (improvement of the motility, the motility rate or the lifespan, reduction in genetic abnormality or the like), the function of sperm obtained from a testis of acting on a female (fertility-improving ability, recurrent miscarriage-improving ability such as deformity-suppressing ability or the like), rejuvenation of any of the testicular functions that has aged with aging (the rejuvenation applies to all the above items) or activation of sperm through addition to semen taken out of the body of an animal.

Here, a testis has Sertoli cells, which are involved in the spermatogenetic function, and Leydig cells, which are involved in the hormone secretion function and release testosterone. In the invention, a testicular function related to the spermatogenetic function is preferably improved.

The diseases on which administration of the testicular function-improving agent of the invention is expected to have an effect or whose risk of onset is expected to be reduced by the administration are types of male infertility, recurrent miscarriage and the like. The types of male infertility are spermatogenesis dysfunction (impaired spermatogenesis), blockage of sperm transport and sexual dysfunction (reproductive dysfunction or erectile function disorder). Of the types of male infertility, the invention can preferably improve male infertility caused by spermatogenesis dysfunction (impaired spermatogenesis) or blockage of sperm transport and can improve fertility caused therefrom. Erectile dysfunction (ED) is excluded from the diseases on which administration of the testicular function-improving agent of the invention is expected to have an effect or whose risk of onset is expected to be reduced by the administration. Moreover, "male infertility caused solely by sexual dysfunction (reproductive dysfunction or erectile function disorder)" is also excluded. Here, erectile dysfunction is caused by reduced function of Leydig cells (which are involved in the hormone secretion function and release testosterone) in a testis and is not directly related to the function of Sertoli cells, which are involved in the spermatogenetic function.

In a preferable aspect, the testicular function-improving agent of the invention is preferably an agent for improving the spermatogenic ability of a testis. The agent for improving the spermatogenic ability of a testis can preferably increase the spermatogenic count when administered to an animal (preferably to a testis of an animal).

In another preferable aspect, the testicular function-improving agent of the invention is preferably a fertility-improving agent. The fertility-improving agent can preferably increase the number of mating with a female or a woman and/or the probability of impregnation (conception rate or spontaneous pregnancy rate) when administered to an animal.

In another preferable aspect, the testicular function-improving agent of the invention is preferably a recurrent miscarriage-improving agent, more preferably a deformity suppressor. The recurrent miscarriage-improving agent is preferably an agent for improving recurrent miscarriage to which a male (or a man) contributes. When administered to an animal, the recurrent miscarriage-improving agent can more preferably increase the number of offspring which can grow normally of the offspring born through mating with a female or a woman, which means that it is more preferable that the abnormal growth rate or the mortality is low. When the recurrent miscarriage-improving agent is administered to an animal, a large number of sperm without genetic abnormality (or with a low rate thereof) are particularly preferably obtained. Moreover, the recurrent miscarriage-improving agent is particularly preferably a deformity suppressor, which means that the administration thereof to an animal can particularly preferably increase the number of offspring without genetic abnormality (or with a low rate thereof).

In this regard, the testicular function-improving agent of the invention can significantly improve the testicular function of an animal and can also be used as a sperm activator which is added to semen taken out of the body of an animal. For example, an aspect in which the testicular function-improving agent of the invention is administered to frozen semen is also included in the testicular function-improving agent of the invention and the method for improving a testicular function. In this regard, however, the testicular function-improving agent of the invention is preferably in a form capable of significantly improving the testicular function of an animal when administered to a testis of the animal. That is, a sperm activator which is added to semen taken out of the body of an animal is preferably excluded from the testicular function-improving agent of the invention.

Compared to the conventional testicular function-improving agents and the conventional sperm activators, the testicular function-improving agent of the invention has advantages such as easiness of mass production, utilization of culture solutions of stem cells which are otherwise disposed of as industrial waste or the like and decreased cost of the disposal of culture solutions of stem cells. In particular, when the culture supernatant of dental pulp-derived stem cells or adipose-derived stem cells is a culture supernatant of human dental pulp-derived mesenchymal stem cells or human adipose-derived stem cells, the testicular function-improving agent of the invention also has advantages of high safety for example from the immunological point of view and less ethical issues in application to a human. When the culture supernatant of dental pulp-derived stem cells or adipose-derived stem cells is a culture supernatant of dental pulp-derived stem cells or adipose-derived stem cells of a patient with any of various diseases (infertility and the like), the application of the testicular function-improving agent of the invention to such a patient will be safer and cause less ethical issues.

The testicular function-improving agent of the invention is derived from a culture supernatant of dental pulp-derived stem cells or adipose-derived stem cells and thus is used also for applications in restorative medicine. In particular, a liquid containing a culture supernatant of dental pulp-derived stem cells or adipose-derived stem cells (particularly SGF) is preferably used for applications in restorative medicine. Here, in regenerative medicine based on stem cell transplantation, it is known that stem cells do not play the main role in regeneration and that liquid components produced by the stem cells repair the organ with the autologous stem cells. Difficult problems of the conventional stem cell transplantation, such as malignant transformation, standardization, the administration method, preservation and the culture method, are solved, and restorative medicine is enabled by the testicular function-improving agent using a culture supernatant of dental pulp-derived stem cells such as SGF or a culture supernatant of adipose-derived stem cells. Compared to stem cell transplantation, restoration using the testicular function-improving agent of the invention does not easily cause neoplastic transformation or the like and is considered to be safer because cell transplantation is not required. Moreover, SGF, a culture supernatant of adipose-derived stem cells and the testicular function-improving agent of the invention have advantages because those with uniformly standardized quality can be used. Because mass production and an efficient administration method can be selected, use with a low cost is enabled.

### [Method for Improving Testicular Function]

The method for improving a testicular function of the invention includes a step of administering the testicular function-improving agent of the invention to an animal.

The method for administering the testicular function-improving agent of the invention to an animal is not particularly restricted.

The administration method is any of infusion, local administration, nasal drops and the like, which are little invasive and with few side effects observed. The local administration method is preferably an injection. Another preferable method is electroporation, in which voltage (electric pulses) is applied to the skin surface to create temporal fine holes in the cell membrane, allowing the active ingredient to penetrate to the dermis layer, which cannot be reached by normal care.

The method for improving a testicular function of the invention preferably includes a step of injecting the testicular function-improving agent of the invention to a testis of the animal.

The testicular function-improving agent of the invention which has been administered to an animal circulates at least in the testes and restores and regenerates any damaged tissue found (including stem cells) through activation of the tissue (stem cells) itself due to the homing effect. Moreover, the administered testicular function-improving agent of the invention sometimes circulates in the body, stimulates the pituitary gland, restores the hormone balance and facilitates the metabolic cycle to the original state.

When administered to semen taken out of the body of an animal, the testicular function-improving agent of the invention activates sperm.

The age (year, week or the like) of the animal as the subject to which the testicular function-improving agent is administered is not particularly restricted. The testicular function-improving agent of the invention may be administered to a young animal or an aged animal.

According to a preferable aspect, the testicular function-improving agent of the invention can improve the testicular function of an aged animal having deteriorated testicular function due to senile weakness or the like and thus is preferably administered to an aged animal in particular. When the testicular function-improving agent of the invention contains microparticles derived from (purified from) a culture supernatant of adipose-derived stem cells, the testicular function of an aged animal having deteriorated testicular function due to senile weakness or the like can be improved to a similar level to that of the testicular function of a young animal. Moreover, when the testicular function-improving agent of the invention contains microparticles derived from (purified from) a culture supernatant of dental pulp-derived stem cells, the testicular function of an aged animal having deteriorated testicular function due to senile weakness or the like can be improved to a same or better level than that of the testicular function of a young animal.

The animal (species) as the subject to which the testicular function-improving agent is administered is not particularly restricted. The animal as the subject to which the testicular function-improving agent is administered is preferably a mammal, a bird (chicken, quail, duck or the like) or fish (salmon, trout, tuna, bonito or the like). The mammal may be a human or a nonhuman mammal but is particularly preferably a human. The nonhuman mammal is more preferably a cow, a pig, a horse, a goat, a sheep, a monkey, a dog, a cat, a mouse, a rat, a guinea pig or a hamster, particularly preferably a horse.

A breed used for racehorses is preferable of the horse breeds, and the Thoroughbred is more preferable. Racehorses are often used as stallions generally when they become old. The testicular function-improving agent of the invention can improve the testicular function (spermatogenic ability or fertility- and recurrent miscarriage-improving ability) of an aged stallion to a same or better level than that of a young stallion, and thus a racehorse is preferable as the animal as the subject to which the testicular function-improving agent of the invention is administered. In some cases, a large amount of semen is obtained from a racehorse while it is still young and stored. Because the testicular function-improving agent of the invention can improve the spermatogenic ability and increase the sperm count of semen, a young racehorse is also preferable as the animal as the subject to which the testicular function-improving agent of the invention is administered.

When the testicular function-improving agent is administered, the dosage of the microparticles per testis is not particularly restricted. In the method for improving a testicular function of the invention, the dosage of the microparticles per testis is preferably 0.1×10⁸ to 1000×10⁸ particles/testis, more preferably 0.5×10⁸ to 100×10⁸ particles/testis, particularly preferably 0.5×10⁸ to 2×10⁸ particles/testis.

### Examples

The invention is explained further specifically referring to Examples and Comparative Examples below. The materials, the amounts, the proportions, the contents of treatments, the procedures of treatments and the like shown in the Examples below can be appropriately changed as long as such changes do not go beyond the intention of the invention. Accordingly, the scope of the invention should not be construed as being limited by the specific examples shown below.

### [Example 1] SGF Exosome

### <Preparation of Culture Supernatant of Dental Pulp-Derived Stem Cells>

A culture supernatant of deciduous dental pulp stem cells was prepared according to the method described in Example 6 of Japanese patent No. 6296622 except that DMEM medium was used instead of the DMEM/HamF12 mixture medium. For the primary culture, fetal bovine serum (FBS) was added to the culture. In the passaging culture, the supernatant was taken from the passaged culture solution which was cultured using the primary culture solution in such a manner that FBS would not be contained, and a culture supernatant of deciduous dental pulp stem cells was thus prepared and used. Here, DMEM is Dulbecco's modified Eagle's medium, and F12 is Ham's F12 medium.

The obtained culture supernatant of deciduous dental pulp stem cells was used as SGF.

### <Preparation of Exosomes from Culture Supernatant of Dental Pulp-Derived Stem Cells>

Exosomes of dental pulp-derived stem cells were purified from the SGF by the following method.

After filtering the culture supernatant of deciduous dental pulp stem cells SGF (100 mL) with a filter having a pore size of 0.22 micrometers, the solution was centrifuged for 60 minutes at 4°C at 100000× g. The supernatant was decanted, and the exosome-concentrated pellets were re-suspended in phosphate-buffered saline (PBS). The re-suspended sample was centrifuged for 60 minutes at 100000× g. The pellets were again recovered from the bottom of the centrifuge tube as the concentrated sample (about 100 µl). The protein concentration was determined using micro BSA protein assay kit (Pierce, Rockford, IL). The exosome-concentrated solution was stored at -80°C.

The obtained exosome-concentrated solution was used as the testicular function-improving agent of Example 1. The testicular function-improving agent of Example 1 is also called SGF exosome below.

### <Characteristics of SGF Exosome>

The average particle size of the microparticles contained in the SGF exosome was 50 to 150 nm.

The SGF exosome contained 2×10⁸ microparticles per 50 µL. This means that the SGF exosome was a highly concentrated exosome solution of 0.04×10⁸ particles/µL.

The components of the obtained SGF exosome were analyzed by a known method. The results showed that the SGF exosome did not contain any dental pulp-derived stem cells, did not contain MCP-1 and did not contain Siglec-9. It was thus found that an active ingredient which is different from MCP-1 and Siglec-9, which are active ingredients of a culture supernatant of mesenchymal stem cells, and from analogs thereof is the active ingredient of the SGF exosome.

### <Spermatogenesis Test in Mice>

To the testes of aged male mice (60 weeks old), 50 µL of the SGF exosome was administered by injection. This means that the SGF exosome containing 2×10⁸ exosomes (20 µg) was directly administered per testis of an aged male mouse.

The testes (caudal epididymides) were taken out two weeks after the administration. The sperm numbers per testis were counted, and an average of two counts was determined. The obtained results are shown in Fig. 1.

### [Example 2] AT-MSC Exosome

A culture supernatant of adipose-derived stem cells was prepared in the same manner as in Example 1 except for using adipose-derived stem cells. Exosomes were prepared from the obtained culture supernatant of adipose-derived stem cells in the same manner as in Example 1. The obtained exosomes are called AT-MSC exosome.

A spermatogenesis test in mice was conducted in the same manner as in Example 1 except for using the AT-MSC exosome. The results obtained are shown in Fig. 1.

### <Characteristics of AT-MSC Exosome>

The average particle size of the microparticles contained in the AT-MSC exosome was 50 to 150 nm.

The AT-MSC exosome contained 2×10⁸ microparticles per 50 µL. This means that the AT-MSC exosome was a highly concentrated exosome solution of 0.04×10⁸ particles/µL.

The components of the obtained AT-MSC exosome were analyzed by a known method. The results showed that the AT-MSC exosome did not contain any adipose-derived stem cells, did not contain MCP-1 and did not contain Siglec-9. It was thus found that an active ingredient which is different from MCP-1 and Siglec-9, which are the active ingredients of a culture supernatant of mesenchymal stem cells, and from analogs thereof is the active ingredient of the AT-MSC exosome.

### [Comparative Example 1] Untreated Control

As an untreated control, a spermatogenesis test in a mouse was conducted in the same manner as in Example 1 using an aged male mouse (60 weeks old) to which the exosomes were not administered (N=1). The results obtained are shown in Fig. 1.

### [Reference Example 1] 12-Week-Old Mouse

A spermatogenesis test in a mouse was conducted in the same manner as in Example 1 using a young male mouse (12 weeks old) to which the exosomes were not administered (N=1). The results obtained are shown in Fig. 1.

First, Fig. 1 shows that, without any treatment (without the administration of the exosomes), the sperm count of the young male mouse (12 weeks old) of Reference Example 1 was higher than that of the aged male mouse of Comparative Example 1.

Next, comparison among the aged male mice (60 weeks old) shows that the sperm count significantly increased with the administration of the SGF exosome of Example 1 or the AT-MSC exosome of Example 2 compared to that of Comparative Example 1 without the administration of the exosomes. In particular, it was found that the administration of the SGF exosome of Example 1 increased the sperm count more and had a higher spermatogenic ability than the administration of the AT-MSC exosome of Example 2.

Comparison between the aged male mice (60 weeks old) and the young male mouse (12 weeks old) shows that, when the AT-MSC exosome of Example 2 was administered, the sperm count increased (recovered) to the same or slightly inferior level to that of Reference Example 1, in which the exosomes were not administered to the young male mouse (12 weeks old). It was found that, especially when the SGF exosome of Example 1 was administered to the aged male mice (60 weeks old), the sperm count significantly increased (enhanced) to the level exceeding the sperm count of Reference Example 1, in which the exosomes were not administered to the young male mouse (12 weeks old). That the administration of the SGF exosome of Example 1 can increase the sperm count of an aged male mouse to exceed the sperm count of a young male mouse is a significant effect which is unexpected to one skilled in the art.

From the above results, it was found that the testicular function-improving agent of the invention can significantly improve the testicular function of an animal in terms of the spermatogenic ability.

### [Examples 11 and 12 and Comparative Examples 11 and 12]

In accordance with the scheme in Fig. 2, a mating test and a birth test were conducted.

Four aged male mice (58 weeks old) were prepared and divided into two groups.

As shown in Fig. 2(B), the SGF exosome obtained in Example 1 was directly administered to the testes of two aged male mice each at 2×10⁸ particles per testis. The dosage was 50 µL per testis. The aged male mouse of Example 11 was named Male 1, and the aged male mouse of Example 12 was named Male 2.

As shown in Fig. 2(A), as a control, physiological saline (PBS control) in the same volume of 50 µL was administered to the testes of two aged male mice instead of the SGF exosome in Examples 11 and 12. The aged male mouse of Comparative Example 11 was named Male 3, and the aged male mouse of Comparative Example 12 was named Male 4.

The aged male mice of Examples 11 and 12 and Comparative Examples 11 and 12 were reared for two weeks.

### <Mating Test with Female Mice>

The vulvae of normal female mice were observed, and female mice with swollen red vulvae (in the proestrus stage in the estrous cycle) were selected and used for mating with the aged male mice of the Examples and the Comparative Examples.

Two young 12-week-old female mice (distinguished by naming the mice Female 1 and Female 2) were cohabited with each of the aged male mice of the Examples and the Comparative Examples, and rearing was started from the evening.

Sixteen hours (next day) and 40 hours (day 2) after starting rearing, the vulvae of the female mice were observed to check whether there was a plug (vaginal plug) and see whether mating occurred. It was determined that mating occurred when a plug was "observed". When a plug was "observed" after 16 hours, observation of a plug was not conducted after 40 hours ("-" in Table 1 and Table 2 below).

The results show that a plug was "observed" in three of the four female mice mated with Male 1 of Example 11 and Male 2 of Example 12, to which the SGF exosome was administered. On the other hand, a plug was "not observed" in all the four female mice mated with Male 3 of Comparative Example 11 and Male 4 of Comparative Example 12 as the PBS control (the exosomes were not administered).

Of the results obtained, the results of mating between Male 1 to Male 4 and Females 1 are shown in Table 1 below, and the results of mating between Male 1 to Male 4 and Females 2 are shown in Table 2 below.

**[Table 1]**

| | **Time of Cohabitation (Mating) with First Female (Female 1)** | |
|---|---|---|
| | Plug after 16 Hours (Next Day) | Plug after 40 Hours (Day 2) |
| Male 1 SGF Exosome | Not Observed | Observed |
| Male 2 SGF Exosome | Observed | - |
| Male 3 PBS Control | Not Observed | Not Observed |
| Male 4 PBS Control | Not Observed | Not Observed |

**[Table 2]**

| | **Time of Cohabitation (Mating) with Second Female (Female 2)** | |
|---|---|---|
| | Plug after 16 Hours (Next Day) | Plug after 40 Hours (Day 2) |
| Male 1 SGF Exosome | Not Observed | Not Observed |
| Male 2 SGF Exosome | Observed | - |
| Male 3 PBS Control | Not Observed | Not Observed |
| Male 4 PBS Control | Not Observed | Not Observed |

### <Birth Test>

Thereafter, the female mice in which mating occurred were reared individually. The birth (delivery) within 20 days was observed, and the numbers of mouse pups were counted (see observation of birth in Fig. 1).

As a result, 15 mouse pups in total were born to the female mice in which a plug was "observed" in Table 1 and Table 2 above, and no mouse pup was born to the female mice in which a plug was "not observed".

Specifically, through mating with Male 1 of Example 11, to which the SGF exosome was administered, four mouse pups were born to Female 1, in which a plug was "observed" (see Table 1 above), and no mouse pup was born to Female 2, in which a plug was "not observed" (see Table 2 above).

Through mating with Male 2 of Example 12, to which the SGF exosome was administered, six mouse pups were born to Female 1, in which a plug was "observed" (see Table 1 above), and five mouse pups were born to Female 2, in which a plug was "observed" (see Table 2 above).

On the other hand, through mating with Male 3 of Comparative Example 11 as the PBS control (the exosomes were not administered), no mouse pup was born to Female 1, in which a plug was "not observed" (see Table 1 above), or to Female 2, in which a plug was "not observed" (see Table 2 above).

Through mating with Male 4 of Comparative Example 12 as the PBS control (the exosomes were not administered), no mouse pup was born to Female 1, in which a plug was "not observed" (see Table 1 above), or to Female 2, in which a plug was "not observed" (see Table 2 above).

Then, 24 hours after the birth, whether there was a milk spot (storage of milk) on the abdomens of the mouse pups was observed. Of the 15 mouse pups born, milk spots were observed in 14 pups. No milk spot was observed in one mouse pup born after mating between Male 2 of Example 12, to which the SGF exosome was administered, and the second female mouse (Female 2).

Of the results obtained, the results of birth after mating between Male 1 to Male 4 and the first females (Females 1), which is shown in Table 1 above, are summarized in Table 3 below, and the results of birth after mating between Male 1 to Male 4 and the second females (Females 2), which is shown in Table 2 above, are summarized in Table 4 below.

**[Table 3]**

| | **Results of Birth after Mating with First Female (Female 1)** | |
|---|---|---|
| | Number of Mouse Pups | Milk Spot (After 24 Hours) |
| Male 1 SGF Exosome | 4 | 4 pups |
| Male 2 SGF Exosome | 6 | 6 pups |
| Male 3 PBS Control | 0 | 0 |
| Male 4 PBS Control | 0 | 0 |

**[Table 4]**

| | **Results of Birth after Mating with Second Female (Female 2)** | |
|---|---|---|
| | Number of Mouse Pups | Milk Spot (After 24 Hours) |
| Male 1 SGF Exosome | 0 | 0 |
| Male 2 SGF Exosome | 5 | 4 |
| Male 3 PBS Control | 0 | 0 |
| Male 4 PBS Control | 0 | 0 |

As shown in Table 1 to Table 4 above, neither of the male mice of Comparative Examples 11 and 12 as the PBS control (the exosomes were not administered), which correspond to normal aged male mice (58 weeks old), could impregnate the female mice. This is believed to be because the testicular function of the male mice was deteriorated due to senile weakness.

On the other hand, the male mice of Example 11 and Example 12, to which the SGF exosome was administered, could each impregnate at least one female mouse, and birth was observed from the female mice. It was thus found that the male mice to which the SGF exosome was administered impregnated the female mice more easily and allowed easy birth of mouse pups.

From the above results, it was found that the testicular function-improving agent of the invention can significantly improve (recover) the testicular function of an animal in terms of the fertility (the ability of impregnating a female).

### <Growth Test of Mouse Pups>

The 15 mouse pups born in the birth test were raised, and the growth state at the age of four weeks was observed.

As a result, 13 of the 15 mouse pups born grew normally, and two pups died (abnormal growth).

Of the results obtained, the results of the growth of the mouse pups born through mating between Male 1 to Male 4 and the first females (Females 1) are shown in Table 5 below, and the results of the growth of the mouse pups born through mating between Male 1 to Male 4 and the second females (Females 2) are shown in Table 6 below.

**[Table 5]**

| | **Results of Growth of Mouse Pups through Mating with First Female (Female 1)** | | |
|---|---|---|---|
| | Observation at Age of 4 Weeks | | |
| | Number of All Mouse Pups | Normal Growth | Abnormal Growth |
| Male 1 SGF Exosome | 4 | 3 | 1 (died, 7 days) |
| Male 2 SGF Exosome | 6 | 6 | 0 |
| Male 3 PBS Control | 0 | 0 | 0 |
| Male 4 PBS Control | 0 | 0 | 0 |

**[Table 6]**

| | **Results of Growth of Mouse Pups through Mating with Second Female (Female 2)** | | |
|---|---|---|---|
| | Observation at Age of 4 Weeks | | |
| | Number of All Mouse Pups | Normal Growth | Abnormal Growth |
| Male 1 SGF Exosome | 0 | 0 | 0 |
| Male 2 SGF Exosome | 5 | 4 | 1 (died, 2 days) |
| Male 3 PBS Control | 0 | 0 | 0 |
| Male 4 PBS Control | 0 | 0 | 0 |

From Table 5 and Table 6 above, it was found that, when the mouse pups were born to the male mouse of Example 11 or Example 12, to which the SGF exosome was administered, the probability of normal growth was high. It was thus found that a male mouse to which the SGF exosome is administered achieves a high probability of mouse pups growing normally and can improve (or suppress) recurrent miscarriage in which the male contributes. The results suggest that, from the male mouse of Example 11 or Example 12, to which the SGF exosome was administered, a large number of sperm without genetic abnormality (or with a low rate thereof) could be obtained, and mouse pups without genetic abnormality (or with a low rate thereof) were born.

In this regard, the growth test could not be conducted using the male mouse of Comparative Example 11 or 12 as the PBS control (the exosomes were not administered), because no mouse pup was born in the birth test.

From the above results, it was found that the testicular function-improving agent of the invention can improve recurrent miscarriage to which a male contributes.

The Examples above show that the testicular function-improving agent of the invention can significantly improve the testicular function of an animal and especially can improve spermatogenic ability, fertility and recurrent miscarriage. In particular, it was found that the testicular function-improving agent of the invention can significantly improve spermatogenic ability, fertility and recurrent miscarriage related to an aged male having deteriorated testicular function (or the reproductive capability) due to senile weakness or the like in a preferable aspect.

### [Example 21] Immortalized MSC Exosome

To human dental pulp-derived stem cells prepared by the same method as in Example 1, a combination of a TGFβ receptor inhibitor, 3-(6-methylpyridin-2-yl)-4-(4-quinolyl)-1-phenylthiocarbamoyl-1H-pyrazole (A-83-01) (Merck), and a ROCK inhibitor, Y-27632 (FUJIFILM Wako Pure Chemical Corporation), was added, and the cells were cultured by a known method. Thus, immortalized MSCs (mesenchymal stem cells) were obtained.

A culture supernatant of the immortalized MSCs was prepared in the same manner as in Example 1 except for using the obtained immortalized MSCs. Exosomes were prepared from the obtained culture supernatant of immortalized MSCs in the same manner as in Example 1. The obtained exosomes are called immortalized MSC exosome.

A spermatogenesis test was conducted in the same manner as in Example 1 except for using the immortalized MSC exosome. Specifically, the immortalized MSC exosome was administered by injection to the testes of an aged male mouse (60 weeks old, N=1). This means that the immortalized MSC exosome containing 2×10⁸ exosomes (20 µg) was directly administered per testis of the aged male mouse.

The testes (caudal epididymides) were taken out two weeks after the administration, and the sperm count per testis was 11.9×10⁶ sperm/caudal epididymis.

### [Comparative Example 21] Untreated Control

As an untreated control, a spermatogenesis test in a mouse was conducted in the same manner as in Example 21 using an aged male mouse (60 weeks old) to which the exosomes were not administered (N=1).

The testes (caudal epididymis) were taken out two weeks after the administration, and the sperm count per testis was 6.3×10⁶ sperm/caudal epididymis.

Example 21 and Comparative Example 21 above show that the sperm count increased significantly when the immortalized MSC exosome of Example 21 was administered compared to that of Comparative Example 21, to which the exosomes were not administered. The above results show that the testicular function-improving agent of the invention can significantly improve the testicular function of an animal in terms of the spermatogenic ability.

## Claims

1. A testicular function-improving agent containing microparticles derived from a culture supernatant of dental pulp-derived stem cells, adipose-derived stem cells or immortalized stem cells thereof,
wherein the improvement of a testicular function is improvement of the ability of a testis itself to form sperm, improvement of the function of sperm themselves obtained from a testis, improvement of the function of sperm obtained from a testis of acting on a female, rejuvenation of any of the functions that has aged with aging or activation of sperm through addition to semen taken out of the body of an animal.

2. The testicular function-improving agent according to claim 1, wherein the microparticles are exosomes.

3. The testicular function-improving agent according to claim 1 or 2
which does not contain the dental pulp-derived stem cells, the adipose-derived stem cells and the immortalized stem cells,
does not contain MCP-1 and
does not contain Siglec-9.

4. The testicular function-improving agent according to any one of claims 1 to 3 which is an agent for improving the spermatogenic ability of a testis.

5. The testicular function-improving agent according to any one of claims 1 to 3 which is a fertility-improving agent.

6. The testicular function-improving agent according to any one of claims 1 to 3 which is a deformity suppressor.

7. The testicular function-improving agent according to any one of claims 1 to 6, wherein the microparticles are microparticles purified from the culture supernatant.

8. The testicular function-improving agent according to any one of claims 1 to 7 which contains 0.5×10⁸ particles or more of the microparticles.

9. The testicular function-improving agent according to any one of claims 1 to 8, wherein the dental pulp-derived stem cells or the adipose-derived stem cells are human dental pulp-derived stem cells or human adipose-derived stem cells.

10. The testicular function-improving agent according to any one of claims 1 to 8, wherein the dental pulp-derived stem cells or the adipose-derived stem cells are dental pulp-derived stem cells of a nonhuman animal or adipose-derived stem cells of a nonhuman animal.

11. A method for improving a testicular function including a step of administering the testicular function-improving agent according to any one of claims 1 to 10 to an animal.

12. The method for improving a testicular function according to claim 11, **characterized by**
improving the spermatogenic ability of a testis of the animal,
improving the fertility of the animal or
suppressing deformity of offspring of the animal.

13. The method for improving a testicular function according to claim 11 or 12, wherein the testicular function-improving agent is injected to a testis of the animal.

14. The method for improving a testicular function according to any one of claims 11 to 13, wherein the dosage of the microparticles per testis of the animal is 0.5×10⁸ to 2×10⁸ particles/testis.
